# EUROPEAN PATENT APPLICATION

(11) **EP 4 074 253 A1**
(43) Date of publication of application: **19.10.2022**
(21) Application number: 21167885.9
(22) Date of filing: 12.04.2021
(51) Int. Cl.: A61B 5/0536, G01R 33/24, G01R 33/56

(54) **SEGMENTATION USING MULTIPLE IMAGE CONTRASTS**

(71) Applicant: Koninklijke Philips N.V., 5656 AG Eindhoven (NL)
(72) Inventor: KATSCHER, Ulrich, 5656 AE Eindhoven (NL); KEUPP, Jochen, 5656 AE Eindhoven (NL)
(74) Representative: Philips Intellectual Property & Standards

(57) **Abstract**

Disclosed herein is a medical system (100, 300) comprising: a memory (110) storing machine executable instructions (120) and a computational system (104). The execution of the machine executable instructions causes the computational system to: receive (200) k-space data, wherein the k-space data encodes multiple magnetic resonance images (124) of a region of interest (309) of a subject (308), wherein the k-space data encodes a B1 field phase measurement, wherein the k-space data encodes a different T1 and/or T2 weighting in each of the multiple magnetic resonance images, reconstruct (202) the multiple magnetic resonance images from the k-space data; reconstruct (204) a measured B1 phase map (126, 700, 702, 704, 706, 708, 710) of the region of interest from a phase map of at least one of the multiple magnetic resonance images; and provide (206) an image segmentation (12) that divides the multiple magnetic resonance images into distinct anatomical regions using a magnitude of the multiple magnetic resonance images, wherein the measured B1 phase map matches the image segmentation.

## Description

### FIELD OF THE INVENTION

The invention relates to magnetic resonance image, in particular to magnetic resonance based electrical properties tomography.

### BACKGROUND OF THE INVENTION

Magnetic resonance imaging (MRI) scanners rely on a large static magnetic field (B0) to align the nuclear spins of atoms as part of the procedure for producing images within the body of a patient. These images can reflect various quantities or properties of the subject. One imaging technique is Electrical Properties Tomography (EPT).

The review article Katscher and van den Berg, "Electric properties tomography: Biochemical, physical and technical background, evaluation and clinical applications," NMR in Biomedicine 2017;e:2729 (DOI: 10.1002/nmb.3729) discloses forward based and other methods of electrical properties tomography (EPT).

### SUMMARY OF THE INVENTION

The invention provides for a medical system, a computer program, and a method in the independent claims. Embodiments are given in the dependent claims.

When performing quantitative magnetic resonance imaging techniques such as EPT a detailed knowledge of the B1 phase is beneficial. If a B1 phase map is segmented correctly, then the B1 phase map can be used to calculate the spatially dependent conductivity within the subject. A difficulty is that often times correct segmentation of the B1 phase map is not available, or the segmentation may not match the B1 phase map because of subject motion.

Embodiments may provide for an improved means of providing a B1 phase map with a segmentation. To achieve this, k-space data that encodes both a B1 phase map and multiple magnetic resonance images is used. The multiple magnetic resonance images have differing T1 and/or T2 weightings. This causes various tissues within a region of interest to have different contrasts. Combining the segmentation of all of the multiple magnetic resonance images provides an accurate segmentation. Because the B1 phase map is encoded in the same data the segmentation automatically applies also to B1 phase map.

In one aspect the invention provides for a medical system that comprises a memory storing machine-executable instructions and a computational system. The computational system may for example be a computational system that is integrated into a magnetic resonance imaging system or it may be another computing type device such as a server or even a web-based or cloud-based computational system. The computational system is intended to represent one or more computational systems at one or more locations.

Execution of the machine-executable instructions causes the computational system to receive k-space data. The k-space data encodes multiple magnetic resonance images of a region of interest of a subject. The k-space data may be provided in several different formats. For example, the k-space data may represent multiple acquisitions of k-space data for each of the individual magnetic resonance images. This could be for k-space data that is acquired one after the other so that the images are essentially acquired in a sequence. In other examples the data could be interleaved. Portions of the k-space data for each of the multiple magnetic resonance images are acquired sequentially and essentially all of the k-space data for the magnetic resonance images are acquired together. The k-space data encodes a B1 field phase measurement.

The k-space data encodes a different T1 and/or T2 weighting in each of the multiple magnetic resonance images. The encoding of each of the multiple magnetic resonance images using a different T1 and/or T2 weighting has the consequence that the different organs and/or tissue types will have different brightnesses relative to each other in the different images. A selection of T1 and T2 weighting is therefore often times referred to as having different contrasts. The encoding using different T1 and T2 weightings cause a different contrast between different tissue types in difference of the images. This may for example facilitate a segmentation.

Execution of the machine-executable instructions further causes the computational system to reconstruct the multiple magnetic resonance images from the k-space data. Execution of the machine-executable instructions further causes the computational system to reconstruct a measured B1 phase map of the region of interest from the B1 field phase measurement of at least one of the multiple magnetic resonance images.

Execution of the machine-executable instructions further causes the computational system to provide an image segmentation of the multiple magnetic resonance images into distinct anatomical regions using a magnitude of the multiple magnetic resonance images. The multiple magnetic resonance images were encoded using different T1 and T2 weightings. The consequence of this is that the different tissues and tissue types within the subject will have different contrasts within each of these different images. The combination of these different images can then be used to provide the image segmentation accurately.

For example, the boundaries between the different tissues may be more apparent in different images for different adjacent tissues or organ type. The measured B1 phase map matches the image segmentation. That is to say that the image segmentation is also a segmentation of the measured B1 phase map. The measured B1 phase map may have a voxel-to-voxel correspondence to the image segmentation. Because the measured B1 phase map matches the image segmentation, there is automatically a registration between the two.

This embodiment may be beneficial because it provides for an accurate segmentation of the B1 phase map. This is useful amongst other things, for performing magnetic resonance electrical properties tomography.

In another embodiment execution of the machine-executable instructions further causes the computational system to calculate an electrical properties tomography map from the measured B1 phase map and the image segmentation. The electrical properties tomography map comprises a conductivity map. The conductivity map can be calculated from the measured B1 phase map, however, normally during these calculations it should be known where the boundaries between different tissue types are. This embodiment may be beneficial because of the accurate segmentation of the measured B1 phase map, the resulting electrical properties tomography map or in this case, the conductivity map may therefore be more accurate.

In another embodiment the electrical properties tomography map is calculated by performing an iterative calculation that assumes a uniform value for the electrical properties tomography map for each of the distinct anatomical regions. The iterative calculations are further performed by calculating a trial B1 field from the uniform value for each of the distinct anatomical regions, and then adjusting the uniform value for each of the distinct anatomical regions using a difference between the measured B1 map and the trial B1 map. In this example the conductivity or the electrical properties tomography map are assumed to be constant within the various segmentations of the multiple magnetic resonance images or the conductivity map. To perform this calculation in this embodiment the value of each of the reasons is assumed to have a constant value and this is used to then calculate a trial B1 field. The error in the trial B1 field can be used iteratively to adjust the trial B1 map until it converges to a solution.

In another embodiment the electrical properties tomography map is calculated globally from the B1 data using Helmholtz's law using image segmentation to define boundaries between regions with a uniform value.

In another embodiment the k-space data further encodes a B1 field magnitude measurement. Execution of the machine-executable instructions further causes the computational system to reconstruct a measured B1 field magnitude map of the region of interest using the k-space data. The calculation of electrical properties tomography map further comprises calculating a conductivity map from the measured B1 field magnitude map and the image segmentation. This embodiment may be beneficial because it also enables the calculation of the conductivity map very accurately. Potential benefits of calculating the B1 magnitude measurement may also include: (1) increasing accuracy of reconstructed conductivity map and (2) enabling the reconstruction of a permittivity map in addition to a conductivity map.

In another embodiment the segmentation comprises an edge identification map for each of the multiple magnetic resonance images and provides the segmentation by overlaying the edge identification map for each of the multiple magnetic resonance images together. For example, the use of edge detection algorithms such as the Sobel algorithm can be used to identify an edge in an image. Using this technique may be particularly effective because each of the multiple magnetic resonance images has a different contrast between the different tissues. One image may show a large degree of contrast between two types of tissues but may have a very low difference in contrast in a different image. In examples, the edge detection from the images are pooled together. This may better help to identify the segmentation correctly.

In another embodiment the medical system further comprises a magnetic resonance imaging system. The memory further contains pulse sequence commands configured to control the magnetic resonance imaging system to acquire the k-space data. The pulse sequence commands are further configured to encode the multiple magnetic resonance images in the k-space data. The pulse sequence commands are further configured to encode a B1 field phased measurement in the k-space data. The pulse sequence commands are further configured to encode a different T1 and/or T2 weighting in each of the multiple magnetic resonance images.

Execution of the machine-executable instructions further causes the computational system to acquire the k-space data by controlling the magnetic resonance imaging system with the pulse sequence commands.

In another embodiment the pulse sequence commands are B1 mapping sequence commands. The B1 mapping sequence commands could for example be configured to encode a B1 magnitude measurement by a double-angle method, a double-TR method, or a Bloch-Siegert shift.

In another embodiment the pulse sequence commands are spin echo pulse sequence commands. The spin echo pulse sequence commands could for example be configured to encode a B1 phase measurement. The different T1 and T2 weightings can be encoded by varying the TR pulse repetition time and/or TE, the echo time in the spin echo pulse sequence commands.

In another embodiment the pulse sequence commands are a balanced gradient echo pulse sequence command. The weighting of the multiple magnetic resonance images with different T1 and/or T2 weighting values can again be by varying the TE and TR times.

In another embodiment, the pulse sequence commands are Inversion-Recovery balanced Turbo-Field Echo (IR-bTFE) pulse sequence commands, including a RF inversion pulse preceding the RF excitation pulse by an inversion time TI, which can additionally be varied to obtain different T1 weighting.

In another embodiment the pulse sequence commands are configured for encoding the different T1 and T2 weighting values in each of the multiple magnetic resonance images by varying the TE and/or TR values.

In another embodiment the k-space data is divided into separate groups of k-space data. For example, there may be separate acquisitions for each of the images. Each of the separate group corresponds to one of the multiple magnetic resonance images.

In another embodiment the k-space data for each multiple magnetic resonance images is interleaved. This embodiment may have the benefit that the k-space data for each of the multiple magnetic resonance images is acquired for comparable time periods of the other images. They may therefore have the same motion state in all of the multiple magnetic resonance images. This may aid in properly performing the segmentation.

In another embodiment the k-space data encodes a different T1 and/or T2 weighting in each of the multiple magnetic resonance images by varying the inversion time TI.

In another aspect the invention provides for a computer program comprising machine-executable instructions for execution by a computational system controlling a medical system. Execution of the machine-executable instructions causes the computational system to receive k-space data. The k-space data encodes multiple magnetic resonance images of a region of interest of a subject. The k-space data encodes a B1 field phase measurement. The k-space data encodes a different T1 and/or T2 weighting in each of the multiple magnetic resonance images.

Execution of the machine-executable instructions further causes the computational system to reconstruct the multiple magnetic resonance images from the k-space data. Execution of the machine-executable instructions further causes the computational system to reconstruct a measured B1 phase map of the region of interest from a phase map of at least one of the multiple magnetic resonance images. Execution of the machine-executable instructions further causes the computational system to provide an image segmentation that divides the multiple magnetic resonance images into distinct anatomical regions using a magnitude of the multiple magnetic resonance images. Because the measured B1 phase map matches the image segmentation, there is automatically a registration between the two.

In another aspect the invention provides for a method of medical imaging. The method comprises receiving k-space data. The k-space data encodes multiple magnetic resonance images of a region of interest of a subject. The k-space data encodes a B1 field phase measurement. The k-space data encodes a different T1 and/or T2 weighting in each of the multiple magnetic resonance images. The method further comprises reconstructing the multiple magnetic resonance images from the k-space data. The method further comprises reconstructing a measured B1 phase map of the region of interest from a phase map of at least one of the multiple magnetic resonance images. The method further comprises providing an image segmentation that divides the multiple magnetic resonance images into distinct anatomical regions using a magnitude of the multiple magnetic resonance images. Because the measured B1 phase map matches the image segmentation, there is automatically a registration between the two.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electromagnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Medical imaging data is defined herein as being recorded measurements made by a tomographic medical imaging system descriptive of a subject. The medical imaging data may be reconstructed into a medical image. A medical image is defined herein as being the reconstructed two- or three-dimensional visualization of anatomic data contained within the medical imaging data. This visualization can be performed using a computer.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance Imaging (MRI) image or MR image is defined herein as being the reconstructed two- or three-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a medical system;
Fig. 2 shows a flow chart which illustrates a method of using the medical system of claim 1;
Fig. 3 illustrates a further example of a medical system;
Fig. 4 shows a flow chart which illustrates a method of using the medical system of claim 1;
Fig. 5 shows a group of multiple magnetic resonance images with different inversion times;
Fig. 6 shows a collection of edge identification maps;
Fig. 7 shows phase maps for the multiple magnetic resonance images shown in Fig. 5; and
Fig. 8 shows IR-bTFE images (above) and conductivity maps for two different inversion times.

### DESCRIPTION OF EMBODIMENTS

Like numbered elements in these figures are either equivalent elements or perform the same function. Elements which have been discussed previously will not necessarily be discussed in later figures if the function is equivalent.

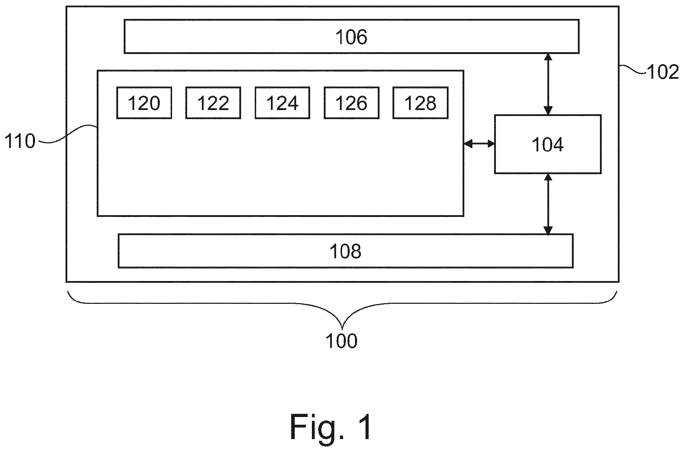

Fig. 1 illustrates an example of a medical system 100. The medical system is shown as comprising a computer 102 that has a computational system 104. The computer 102 is intended to represent one or more computers that may be networked together and that may operate collectively. The computational system 104 is likewise intended to represent one or more computational systems such as one or more processing cores that are distributed in one or more computers 102.

The computer is shown as having an optional hardware interface 106 and an optional user interface 108 that are in communication with the computational system 104. The hardware interface 106 may for example be used for controlling other components of the medical system 100 if they are present. The user interface 108 may for example be used by an operator to control and/or interact with the medical system 100. The medical system 100 is further shown as comprising a memory 110 that is in communication with the computational system 104. The memory 110 is intended to represent one or more different types of memory that are in communication with the computational system 104.

The memory is shown as containing machine-executable instructions 120. The machine-executable instructions 120 enable the computational system 104 to perform various tasks such as data processing, image processing, and controlling other components. The memory 110 is further shown as containing k-space data 122. The k-space data is configured such that it encodes multiple magnetic resonance images. The k-space data is further encoded such that each of the multiple magnetic resonance images has a different T1 and/or T2 weighting. The k-space data 122 further encodes a B1 field phase measurement. The memory 110 is further shown as containing multiple magnetic resonance images 124 that were reconstructed from the k-space data 122. The memory 110 is further shown as containing a measured B1 phase map 126 that was also reconstructed from the k-space data 122. The memory 110 is further shown as containing image segmentation 128 that was reconstructed or determined from the multiple magnetic resonance images 124.

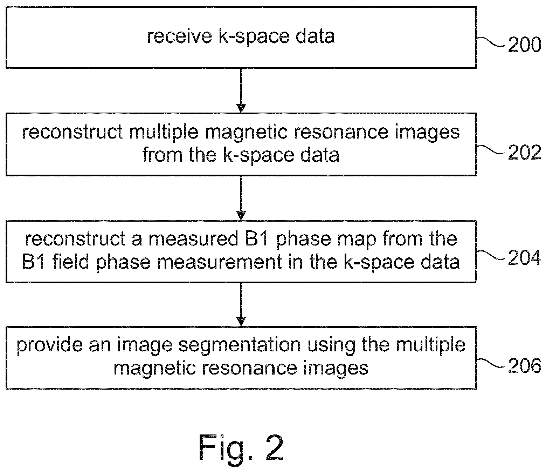

Fig. 2 shows a flowchart which illustrates a method of operating the medical system 100 of Fig. 1. First, in step 200, the k-space data 122 is received. Next, in step 202, the multiple magnetic resonance images 124 are reconstructed from the k-space data 122. Then, in step 204, the measured B1 phase map 126 is also reconstructed from the k-space data 122. Finally, in step 206, the image segmentation 128 is reconstructed from the multiple magnetic resonance images 124.

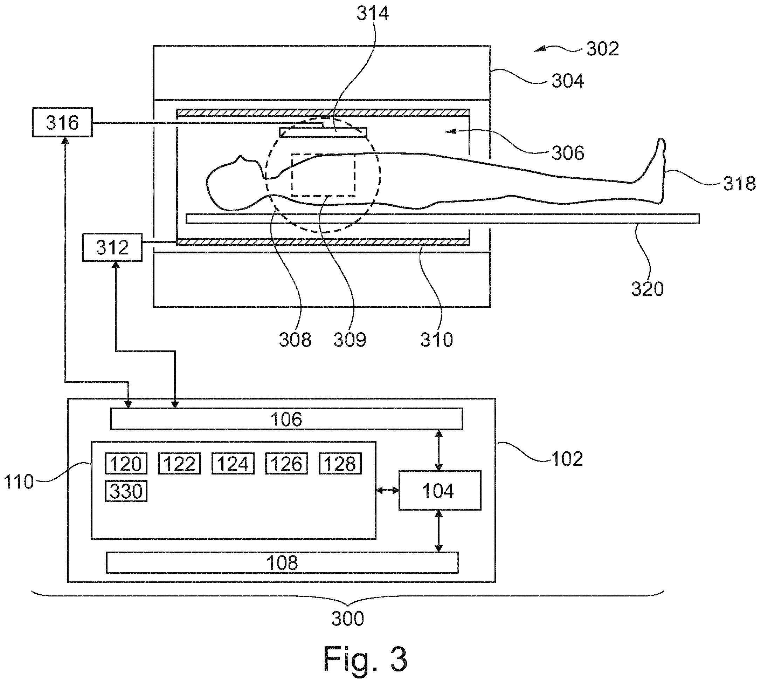

Fig. 3 illustrates a further example of a medical system 300. The medical system 300 in Fig. 3 is similar to the medical system 100 in Fig. 1 except that it additionally comprises a magnetic resonance imaging system 302 that is controlled by the computational system 104.

The magnetic resonance imaging system 302 comprises a magnet 304. The magnet 304 is a superconducting cylindrical type magnet with a bore 306 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A region of interest 309 is shown within the imaging zone 308. The magnetic resonance data that is acquired typically acquried for the region of interest. A subject 318 is shown as being supported by a subject support 320 such that at least a portion of the subject 318 is within the imaging zone 308 and the region of interest 309.

Within the bore 306 of the magnet there is also a set of magnetic field gradient coils 310 which is used for acquisition of preliminary magnetic resonance data to spatially encode magnetic spins within the imaging zone 308 of the magnet 304. The magnetic field gradient coils 310 connected to a magnetic field gradient coil power supply 312. The magnetic field gradient coils 310 are intended to be representative. Typically magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise the transceiver 316 may also represent separate transmitters and receivers. The radio-frequency coil 314 may also have multiple receive/transmit elements and the radio frequency transceiver 316 may have multiple receive/transmit channels. For example if a parallel imaging technique such as SENSE is performed, the radio-frequency could 314 will have multiple coil elements.

The transceiver 316 and the gradient controller 312 are shown as being connected to the hardware interface 106 of the computer system 102.

The memory 110 is further shown as containing pulse sequence commands 330. The pulse sequence commands are commands or data which may be converted into the commands that enable the computational system 104 to control the magnetic resonance imaging system 302 to acquire the k-space data 122. The pulse sequence commands 330 are configured to acquire k-space data 122 that encodes the multiple magnetic resonance images 124 and the measured B1 phase map 126.

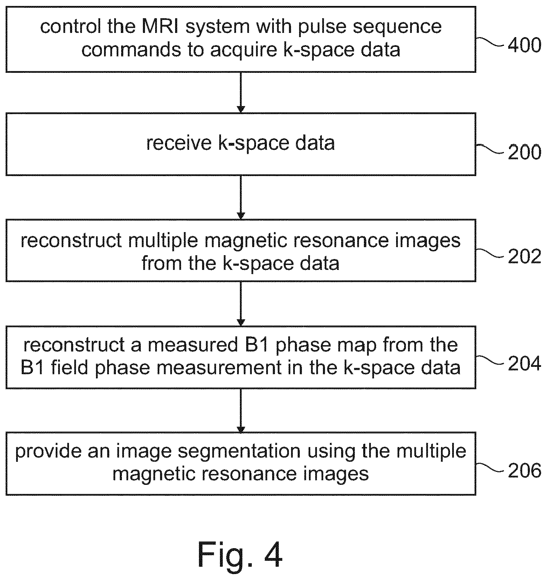

Fig. 4 shows a flowchart which illustrates a method of operating the medical system 300 of Fig. 3. First, in step 400, the computational system 104 controls the magnetic resonance imaging system 302 with the pulse sequence commands 330 and to acquire the k-space data 122. After step 400 the method proceeds to steps 200, 202, 204, and 206 as is illustrated in Fig. 2.

The MR-based reconstruction of conductivity via "Electrical Properties Tomography" (EPT) uses the input of the B1 -phase (measured B1 phase map 126) as well as tissue compartment boundaries (image segmentation 128). Ideally, both inputs are taken from the same scan, such that inter-scan patient motion or potential geometric distortion between different scans are excluded.

One of the most suitable sequences for EPT fulfilling these requirements is the balanced Fast Field Echo (bFFE) sequence. The bFFE sequence however suffers from missing or weak contrast between certain tissue types, which may have distinct electrical properties, such that conductivity reconstruction of certain areas particularly in the brain is difficult or impossible.

Some examples may use Inversion-Recovery balanced Turbo-Field Echo (IR-bTFE) with varying inversion time TI instead of bFFE. Other options are to use spin echo pulse sequence commands or balanced gradient echo pulse sequence commands.

On one hand, this sequence provides a phase map free of B0 components as in the case of bFFE. On the other hand, a wide range of contrasts can be achieved by varying TI, such that the desired tissue boundaries are provided, yielding more reliable conductivity maps.

One example may use Inversion-Recovery balanced Turbo-Field Echo (IR-bTFE) with varying inversion time TI instead of bFFE. On one hand, this sequence provides a phase map free of B0 components as in the case of bFFE. On the other hand, a wide range of contrasts can be achieved by varying TI, such that the desired tissue boundaries are provided, as opposed to bFFE.

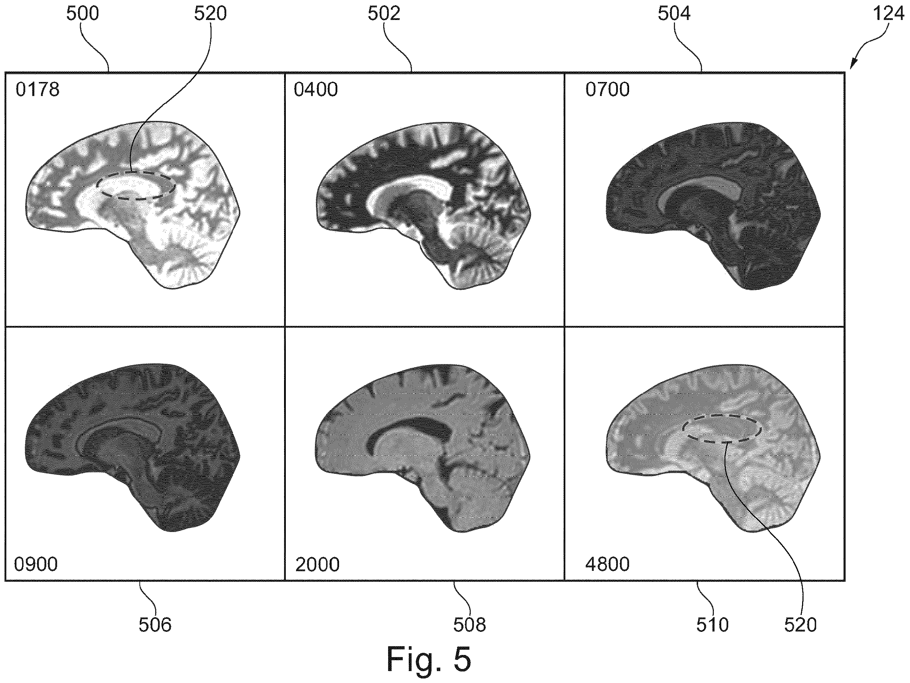

Contrast obtained with IR-bTFE is a complex interplay of TI, TE, and TR. While TE and TR are chosen as short as possible, the variation of TI results in various image contrasts (see Fig. 5 below). It is possible to measure with a single TI (for one particular contrast) or with multiple TIs (to better delineate various tissue types). Measuring with multiple TIs can be performed sequentially or in an interleaved manner.

Fig. 5 shows a group of multiple magnetic resonance images 124. The images 124 depicted in Fig. 5 have their TI or inversion time varied from 178 ms to 4800 ms. Image 500 has a TI of 178 ms. Image 502 has a TI of 400 ms. Image 504 has a TI of 700 ms. Image 506 has a TI of 900 ms. Image 508 has a TI of 2000 ms. Image 510 has a TI of 4800 ms. It can be seen that in each of these images the contrast of the different tissue types is different. Each of the images shows differing information about the boundaries between various anatomical structures and tissues.

Fig. 5 shows brain images of a volunteer using six different values of TI, leading to different contrasts. As example, an almost vanishing contrast is found between compartments of cerebrospinal fluid (CSF) and gray matter compartments (i.e., cortical gray matter or deep gray matter nuclei) for low TI, corresponding to bFFE. In Fig. 5 the example of the caudate nucleus 520 is annotated, which shows insufficient contrast at TI = 178 ms, as it would be the case for bFFE. At TI=4800 ms, a clear contrast between these two compartments is visible.

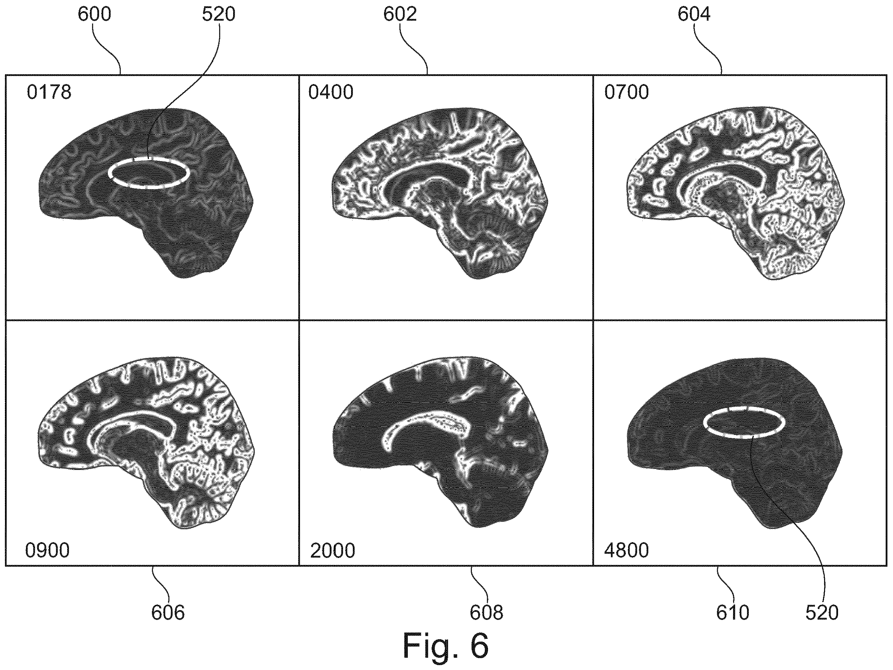

If multiple TIs are used, boundaries can be calculated for each TI separately (see Fig. 6), and the optimum boundary plot can be obtained, e.g., from a maximum intensity projection over all boundary maps.

Fig. 6 shows a collection of edge identification maps 600, 602, 604, 606, 608, 610. Image 600 is the edge identification map for image 500. Image 602 is the edge identification map for image 502. 604 is the edge identification map for image 504. Image 606 is the edge identification map for image 506. Image 608 is the edge identification map for image 508. Image 610 is the edge identification map for image 510. It can be seen in these images that the images 500-510 provided different information about the boundaries between different anatomical regions and tissue types. The edge identification maps 600-610 could for example be found with an edge detection algorithm such as a Sobel filter.

The caudate nucleus region labeled 520 in Fig. 5 illustrates a region where there is a difference in contrast between the ventricle and caudate nucleus. It can be seen that in images 500 and 510 the details which are visible are quite different within the zone 520. The difference in region 520 is also marked on images 600 and 610 of Fig. 6. It can be seen that the edge identification maps in this zone 520 also contain very different information. This region 520 is illustrative how the different images 600-610 can be used to provide a segmentation. The image segmentation 128 could for example be constructed by combining the edge information combined in images 600-610.

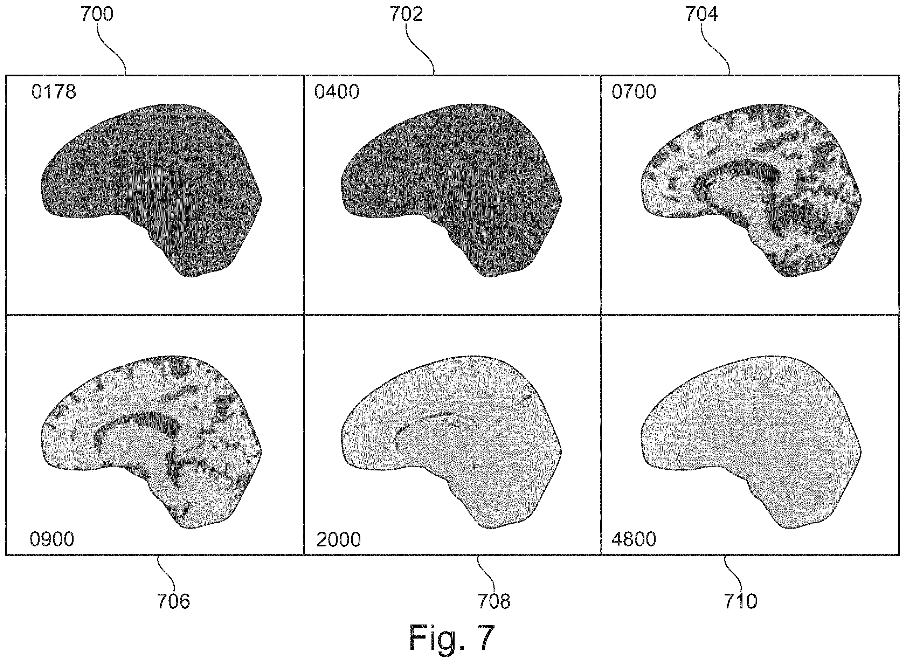

For intermediate TI, phase jumps occur at tissue boundaries with respective T1 and T2 (Fig. 7, grey scaled maps). These phase jumps can be additionally taken into account for boundary detection. It is important to note that apart from these phase jumps (i.e., at low and at high TI), the same (B0-free) phase map is obtained, except a physically irrelevant, constant offset of 180°.

Fig. 7 shows phase maps 700, 702, 704, 706, 708, 710 that correspond to the magnitude images 500-510 shown in Fig. 5. Image 700 is the phase map corresponding to magnitude image 500. Image 702 is the phase map corresponding to magnitude image 502. Image 704 is the phase map corresponding to magnitude image 504. Image 706 is the phase map corresponding to magnitude image 506. Image 708 is the phase map corresponding to magnitude image 508. Image 710 is the phase map corresponding to magnitude image 510. Image 700 and image 710 are essentially the same phase map that are 180° offset.

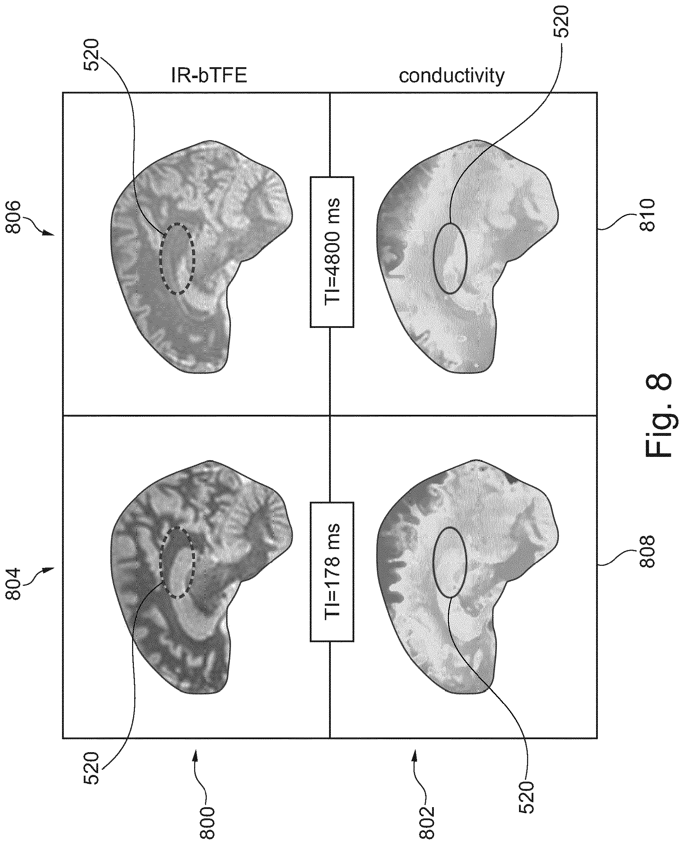

An example for the resulting conductivity maps of low TI (178 ms) and high TI (4800 ms) is shown in Fig. 8. For TI=178 ms, conductivity in the annotated area of caudate nucleus shows a blurred distribution, because of the missing tissue boundary delineation. For TI=4800 ms, conductivity shows a clear difference between ventricle and caudate nucleus as expected, based on the contrast highlighting the actual tissue boundaries. Similar features can be found in other parts of the conductivity maps.

Fig. 8 shows several images. The images in row 800 are IRbTFE images. Below, in row 802, are the corresponding conductivity maps. In column 804 the inversion time is 178 ms. In column 806 the inversion time is 4800 ms. The caudate nucleus region 520 is also illustrated in these images. For the conductivity map 808 with TI=178 ms, conductivity in the annotated area of caudate nucleus shows a blurred distribution between high values close to the ventricle and low values apart from the ventricles. For the conductivity map 810 with TI=4800 ms, conductivity shows a clear difference between ventricle and caudate nucleus as expected.

Examples may provide for improved conductivity mapping in general and in particular, to improve characterization of certain brain regions like deep gray matter nuclei, as of importance e.g. for neurodegeneration like Parkinson's or Alzheimer's disease.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: computational system
- 106: optional hardware interface
- 108: optional user interface
- 110: memory
- 120: machine executable instructions
- 122: k-space data
- 124: multiple magnetic resonance images
- 126: measured B1 phase map
- 128: image segmentation
- 200: receive k-space data
- 202: reconstruct the multiple magnetic resonance images from the k-space data
- 204: reconstruct a measured B 1 phase map of the region of interest from a phase map of at least one of the multiple magnetic resonance images
- 206: provide image segmentation that divides the multiple magnetic resonance images into distinct anatomical regions using a magnitude of the multiple magnetic resonance images
- 300: medical system
- 302: magnetic resonance imaging system
- 304: magnet
- 306: bore of magnet
- 308: imaging zone
- 309: region of interest
- 310: magnetic field gradient coils
- 312: magnetic field gradient coil power supply
- 314: radio-frequency coil
- 316: transceiver
- 318: subject
- 320: subject support
- 330: pulse sequence commands
- 400: acquire the k-space data by controlling the magnetic resonance imaging system with the pulse sequence commands
- 500: Magnitude image with an inversion time (TI) of 178 ms
- 502: Magnitude image with an inversion time (TI) of 400 ms
- 504: Magnitude image with an inversion time (TI) of 402 ms
- 506: Magnitude image with an inversion time (TI) of 404 ms
- 508: Magnitude image with an inversion time (TI) of 406 ms
- 510: Magnitude image with an inversion time (TI) of 408 ms
- 520: caudate nucleus
- 600: edge identification map for image 500
- 602: edge identification map for image 502
- 604: edge identification map for image 504
- 606: edge identification map for image 506
- 608: edge identification map for image 508
- 610: edge identification map for image 510
- 700: phase map corresponding to magnitude image 500
- 702: phase map corresponding to magnitude image 502
- 704: phase map corresponding to magnitude image 504
- 706: phase map corresponding to magnitude image 506
- 708: phase map corresponding to magnitude image 508
- 710: phase map corresponding to magnitude image 510
- 800: IRbTFE images
- 802: conductivity maps
- 804: inversion time of 178 ms
- 806: inversion time of 4800 ms
- 808: conductivity map for 178 ms
- 810: conductivity map for 4800 ms

## Claims

1. A medical system (100, 300) comprising:
- a memory (110) storing machine executable instructions (120);
- a computational system (104), wherein execution of the machine executable instructions causes the computational system to:
- receive (200) k-space data (122), wherein the k-space data encodes multiple magnetic resonance images (124) of a region of interest (309) of a subject (318), wherein the k-space data encodes a B1 field phase measurement, wherein the k-space data encodes a different T1 and/or T2 weighting in each of the multiple magnetic resonance images,
- reconstruct (202) the multiple magnetic resonance images from the k-space data;
- reconstruct (204) a measured B1 phase map (126, 700, 702, 704, 706, 708, 710) of the region of interest from a phase map of at least one of the multiple magnetic resonance images; and
- provide (206) an image segmentation (128) that divides the multiple magnetic resonance images into distinct anatomical regions using a magnitude of the multiple magnetic resonance images, wherein the measured B1 phase map matches the image segmentation.

2. The medical system of claim 1, execution of the machine executable instructions further causes the computational system to calculate an electrical properties tomography map from the measured B1 phase map and the image segmentation, wherein the electrical properties tomography map comprises a conductivity map (808, 810).

3. The medical system of claim 2, wherein the at least one electrical properties tomography map is calculated using any one of the following:
- by performing an iterative calculating that assumes a uniform value for the electrical properties tomography map for each of the distinct anatomical regions, calculates a trial B1 field from the uniform value for each of the distinct anatomical regions, and then adjusts the uniform value for each of the distinct anatomical regions using a difference between the measured B1 map and the trial B1 map; and
- by calculating the electrical properties tomography map globally from the B1 data using Helmholtz's law using the image segmentation to define boundaries between regions with a uniform value for the electrical properties tomography map.

4. The medical system of claim 2 or 3, wherein the k-space data further encodes a B1 field magnitude measurement, wherein execution of the machine executable instructions further causes the computational system to reconstruct a measured B1 field magnitude map of the region of interest using the k-space data, wherein calculation of the electrical properties tomography map further comprises calculating a permittivity map from the measured B1 field magnitude map and the image segmentation.

5. The medical system of any one of claims 1 to 4, wherein the segmentation comprises calculating an edge identification map (600, 602, 604, 606, 608, 610) for each of the multiple magnetic resonance images and provide the segmentation by overlaying the edge identification map for each of the multiple magnetic resonance images together.

6. The medical system of any one of the preceding claims, wherein the medical system further contains a magnetic resonance imaging system (302), wherein the memory further contains pulse sequence commands (330) configured to encode the multiple magnetic resonance images in the k-space data, wherein the pulse sequence commands are configured to encode a B1 field phase measurement in the k-space data, wherein the pulse sequence commands are further configured to encode a different T1 and/or T2 weighting in each of the multiple magnetic resonance images, wherein execution of the machine executable instructions further causes the computational system to acquire (400) the k-space data by controlling the magnetic resonance imaging system with the pulse sequence commands.

7. The medical system of any one of the preceding claims, wherein the pulse sequence commands are spin echo pulse sequence commands.

8. The medical system of any one of claims 1 through 6, wherein the pulse sequence commands are balanced gradient echo pulse sequence commands.

9. The medical system of claim 1 through 6, wherein the pulse sequence commands are Inversion-Recovery balanced Turbo-Field Echo pulse sequence commands.

10. The medical system of any one of claims 5 through 9, wherein the pulse sequence commands are configured for encoding the different T1 and/or T2 weighting in each of the multiple magnetic resonance images by varying TE and/or TR values and/or TI values.

11. The medical system of any one of the preceding claims, wherein the k-space data is divided into separate groups of k-space data, and where each of the separate groups corresponds to one of the multiple magnetic resonance images.

12. The medical system of any one of claims 1 through 10, wherein the k-space data for each of the multiple magnetic resonance images is interleaved.

13. A computer program comprising machine executable instructions (120) for execution by a computational system (104) controlling a medical system (100, 300), wherein execution of the machine executable instructions causes the computational system to:
- receive (200) k-space data (122), wherein the k-space data encodes multiple magnetic resonance images (124) of a region of interest (309) of a subject (318), wherein the k-space data encodes a B1 field phase measurement, wherein the k-space data encodes a different T1 and/or T2 weighting in each of the multiple magnetic resonance images,
- reconstruct (202) the multiple magnetic resonance images from the k-space data;
- reconstruct (204) a measured B1 phase map (126, 700, 702, 704, 706, 708, 710) of the region of interest from a phase map of at least one of the multiple magnetic resonance images; and
- provide (206) an image segmentation (128) that divides the multiple magnetic resonance images into distinct anatomical regions using a magnitude of the multiple magnetic resonance images, wherein the measured B1 phase map matches the image segmentation.

14. A method of medical imaging, wherein the method comprises:
- receiving (200) k-space data (122), wherein the k-space data encodes multiple magnetic resonance images (124) of a region of interest (309) of a subject (318), wherein the k-space data encodes a B1 field phase measurement, wherein the k-space data encodes a different T1 and/or T2 weighting in each of the multiple magnetic resonance images,
- reconstructing (202) the multiple magnetic resonance images from the k-space data;
- reconstructing (204) a measured B1 phase map (126, 700, 702, 704, 706, 708, 710) of the region of interest from a phase map of at least one of the multiple magnetic resonance images; and
- providing (206) an image segmentation (128) that divides the multiple magnetic resonance images into distinct anatomical regions using a magnitude of the multiple magnetic resonance images, wherein the measured B1 phase map matches the image segmentation.
